# DEMANDE DE BREVET EUROPEEN

(11) **EP 1 529 519 A2**
(43) Date de publication de la demande: **11.05.2005**
(21) Numéro de dépôt: 04292590.9
(22) Date de dépôt: 02.11.2004
(51) Int. Cl.: A61K 7/40, A61P 17/10

(54) **Utilisation cosmétique de particules de gel cubique en tant qu'agent empêchant ou réduisant l'adhésion des microorganismes sur la surface de la peau et/ou des muqueuses**

(30) Priorité: 07.11.2003 FR 0350806
(71) Demandeur: L'OREAL, 75008 Paris (FR)
(72) Inventeur: Potin, Anthony, 75006 Paris (FR)
(74) Mandataire: Kromer, Christophe

(57) **Abrégé**

La présente invention se rapporte à l'utilisation cosmétique de particules de gel cubique en tant qu'agent empêchant ou réduisant l'adhésion des microorganismes sur la surface de la peau et/ou des muqueuses.

L'invention concerne également un procédé cosmétique pour prévenir et/ou lutter contre les désordres liés à l'adhésion des micoorganismes liés à l'adhésion des micoorganismes comprenant l'application sur la peau et/ou les muqueuses, d'une composition contenant des particules de gel cubique.

Les particules de gel cubique sont de préférence en dispersion aqueuse et sont formées soit à partir d'un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et d'un agent dispersant et stabilisant, soit par mélange d'au moins deux composés amphiphiles, l'un des composés amphiphiles étant susceptible de former en présence d'eau une phase lamellaire et l'autre étant susceptible de former en présence d'eau une phase hexagonale inverse.

## Description

La présente demande se rapporte à l'utilisation de particules de gel cubique dans une composition cosmétique ou pour la préparation d'une composition dermatologique en tant qu'agent empêchant ou réduisant l'adhésion des microorganismes sur la surface de la peau et/ou des muqueuses.

La peau humaine est peuplée en permanence d'une multitude de microorganismes différents (bactéries, levures et champignons). Les microorganismes commensaux vivant sur ou dans la peau peuvent faire partie d'une microflore soit résidente (normale), soit transitoire. La flore microbienne résidente, indispensable à la bonne santé de la peau, est constituée principalement de staphylocoques (*Staphylococcus epidermis* et *Staphylococcus hominis*), de corynebactéries, de propionibactéries Gram+ telles que *Propionibacterium acnes*, ainsi que d'une flore fongique principalement composée de *Pytosporum ovale.* Leur présence est établie en profils de distribution bien définis. Habituellement, les micro-organismes transitoires ne se fixent pas fermement; ils sont incapables de se multiplier et meurent normalement après quelques heures.

L'anatomie et la physiologie de la peau varient d'une partie à l'autre du corps et la microflore résidante reflète ces variations.

La plupart des bactéries de la peau sont présentes sur l'épiderme squameux superficiel, colonisant les cellules mortes et étroitement associées aux glandes sébacées et sudoripares. Les excrétions de ces glandes fournissent de l'eau, des acides aminés, de l'urée, des électrolytes et des acides gras spécifiques servant d'éléments nutritifs principalement pour *Staphylococcus epidermidis* et des corynébactéries aérobies.

Les infections cutanées sont le plus souvent dues à la rupture de l'équilibre écologique de la flore résidente suite à la colonisation de la peau par des germes exogènes pathogènes ou à la prolifération anormale d'une souche endogène. Les germes pathogènes les plus connus sont *Pseudomonas aeruginosa* (Gram -) qui est responsable de petits boutons, de folliculites, de rougeurs et de prurit, *Candida albicans* pouvant provoquer des inflammations à la commissure des lèvres, candidoses cutanées, du prurit, des folliculites et des aphtes, *Staphylococcus aureus* pouvant provoquer des boutons, des folliculites, de l'impétigo, et des furoncles, et *Streptococcus* du groupe A responsable d'impétigo.

Pour combattre ces micro-organismes, il est courant d'utiliser des antibiotiques ou des bactéricides. L'utilisation de ces composés pose cependant le problème de la non spécificité d'action visant indifféremment la flore pathogène et la flore résidente, le problème du risque d'apparition de résistances bactériennes, ainsi que des problèmes de tolérance cutanée (irritations, allergies, ...).

Il est également connu de réduire ou de prévenir la colonisation de surfaces telles que les dents, la peau et/ou les muqueuses, par des germes pathogènes en empêchant leur fixation sur ces supports. Les composés utilisés en tant qu'agents anti-adhésion décrits dans l'art antérieur sont soit des silicones (WO-99/62475), soit des hydrates de carbone et des dérivés d'hydrates de carbone, tels que ceux décrits dans la demande de brevet WO-96/23479, soit des huiles végétales comme décrit dans la demande de brevet EP-1 133 979.

Or, la plupart des hydrates de carbone constituent une source de carbone pour des bactéries et champignons. Leur présence dans des compositions cosmétiques favorise par conséquent la prolifération microbienne et nécessite l'augmentation de la concentration en agents conservateurs (bactéricides ou bactériostatiques). Cet inconvénient annule ainsi le bénéfice de l'approche consistant à remplacer des composés antibiotiques ou bactéricides par des composés réduisant l'adhérence microbienne.

La demanderesse a mis en évidence que, de façon surprenante, les particules de gel cubique permettaient de réduire de manière significative l'adhésion des microorganismes à la surface de la peau et/ou des muqueuses, et de prévenir ainsi la prolifération de germes potentiellement pathogènes en l'absence d'agent antibiotiques, bactéricides ou fongicides, et sans qu'il soit nécessaire d'utiliser une quantité importante d'agents conservateurs.

Les particules de gel cubique, à la différence des hydrates de carbone qui se lient aux récepteurs des micro-organismes pour empêcher les liaisons aux glycolipides des cornéocytes, agissent sur les propriétés physico-chimiques de la surface de la peau et/ou des muqueuses, ces propriétés physico-chimiques faisant intervenir les interactions électrodynamiques dues aux forces de Van der Waals, les interactions acido-basiques selon Lewis et les interactions électrostatiques.

Le terme de gel cubique désigne des gels transparents, isotropes en lumière polarisée, se présentant sous forme de phase cristal liquide cubique. Les phases cubiques sont organisées d'une manière bi-polaire en domaines hydrophile et lipophile distincts, en contact étroit et formant un réseau tridimensionnel thermodynamiquement stable. Une telle organisation a été notamment décrite dans Luzzati (1968), « Biological Membranes » (Chapman, D. Ed.), vol 1, 71-123 et dans Mariani et coll. (1988), J. Mol. Biol., 204, 165-189, ainsi que dans "La Recherche" (1992), Vol.23, 306-315. Selon l'arrangement des domaines hydrophile et lipophile, la phase cubique est dite de type normal ou inverse. Le terme de gel cubique utilisé selon la présente invention regroupe bien entendu les gels présentant les différents types de phases cubiques.

L'invention a donc pour objet l'utilisation cosmétique non thérapeutique de particules de gel cubique dans une composition en tant qu'agent empêchant ou réduisant l'adhésion des microorganismes sur la surface de la peau et/ou des muqueuses.

L'invention a encore pour objet l'utilisation de particules de gel cubique pour la préparation d'une composition dermatologique destinée prévenir ou lutter contre les pathologies liées à l'adhésion des microorganismes sur la surface de la peau et/ou des muqueuses.

Par "*empêcher ou réduire l'adhésion des micro-organismes*", il faut entendre que les particules de gel cubique ou la composition les contenant peuvent être utilisées aussi bien à titre préventif, pour leur capacité à prévenir, totalement ou partiellement, l'adhésion des micro-organismes, qu'à titre curatif pour leur capacité à faciliter le détachement des micro-organismes.

Les particules de gel cubique peuvent être utilisées seules et constituer la composition à utiliser, ou être incorporées dans une composition et notamment dans une émulsion huile-dans-eau (H/E) ou eau-dans-huile (E/H).

Quand le gel cubique est dispersé dans un milieu aqueux, on obtient des particules de gel cubique en dispersion, particules qui ont la même structure que le gel cubique non dispersé.

Les particules de gel cubique utilisées selon l'invention peuvent être présentes dans la composition pour application topique, en une quantité allant par exemple de 0,1 à 20 % en poids et de préférence de 0,1 à 10 % en poids par rapport au poids total de la composition.

Tous les types de particules de gel cubique peuvent être utilisés selon la présente invention. De manière avantageuse, les particules de gel cubique utilisées sont en dispersion aqueuse. Elles peuvent être notamment obtenues selon les deux modes préférés de réalisation décrits ci-dessous.

Selon un premier mode de réalisation, les particules se trouvent en dispersion aqueuse et sont formées par un mélange comprenant (i) de 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et (ii) de 0,05 à 3 % en poids par rapport au poids total de la composition, d'au moins un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone. Selon ce mode de réalisation des particules de gel cubique utilisées selon l'invention, la proportion pondérale relative en composé (i) par rapport au poids à l'agent dispersant et stabilisant (ii) peut aller par exemple de 2 à 200, et de préférence de 5 à 50.

Le phytantriol utilisable comme composé (i) est un composé connu, notamment commercialisé sous la dénomination de « Phytantriol-63926 » par la Société Roche.

Parmi les dérivés N-2-alcoxycarbonyles de N-méthylglucamine, on peut notamment citer ceux répondant à la formule (I) suivante : 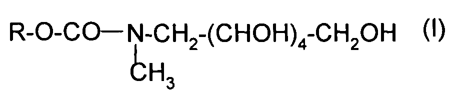 dans laquelle R représente un radical alkyle ramifié comportant de 6 à 18 atomes de carbone.

Parmi ceux-ci, on peut notamment mentionner la N-2-hexyldécyloxycarbonyl-N-méthyl-glucamine, la N-2-éthylhexyloxycarbonyl-N-méthyl-glucamine, la N-2-butyloctyloxycarbonyl-N-méthyl-glucamine et leurs mélanges.

Les composés de formule (I) tels que définis ci-dessus sont décrits et peuvent être préparés selon le procédé décrit dans le document EP-A-711540 cité ici pour référence. Ce procédé comprend notamment les étapes consistant :
(a) à dissoudre dans un mélange d'eau et d'un solvant organique, de la N-méthyl-glucamine, le solvant pouvant être par exemple le tétrahydrofuranne,
(b) à disperser dans le mélange obtenu précédemment, du bicarbonate de sodium, en une proportion appropriée correspondant environ à quatre fois la proportion molaire de N-méthyl-glucamine,
(c) à introduire alors dans le mélange réactionnel obtenu, un chloroformiate d'alkyle, le radical alkyle étant en C6-C18, en une proportion appropriée, généralement équimolaire par rapport à celle de N-méthyl-glucamine, puis à laisser réagir le mélange, et
(d) à filtrer le mélange réactionnel obtenu après l'étape (c), à recueillir le résidu pâteux obtenu par filtration, puis à le dissoudre dans de l'acétone en vue de sa cristallisation à une température de l'ordre de 5°C. Après filtration, les cristaux du dérivé N-2-alcoxycarbonyle de N-méthylglucamine formé sont essorés et séchés sous vide.

Dans le cas où l'on utilise un composé de formule (I) comme composé (i), les particules de gel cubique utilisées selon l'invention contiennent de préférence un mélange d'un tel composé et de phytantriol, et plus précisément un mélange comprenant une quantité de phytantriol allant de 1 à 40 % en poids et mieux de 10 à 30 % en poids par rapport au poids du mélange, et une quantité de dérivé N-2-alcoxycarbonyle de N-méthylglucamine de formule (I) allant de 60 à 99 % en poids et mieux de 70 à 90 % en poids par rapport au poids du mélange.

Les monoglycérides d'acide gras insaturé pouvant être utilisés comme composés (i) dans ce premier mode de réalisation sont de préférence ceux ayant une chaîne grasse insaturée comportant de 16 à 22 atomes de carbone. Parmi ceux-ci, on peut notamment citer le monooléate de glycéryle ou monooléine et le monolinoléate de glycéryle ou monolinoléine. On peut bien entendu utiliser pour préparer les dispersions de particules de gel cubique, un mélange de monoglycérides tels que définis précédemment ainsi qu'un mélange de monoglycérides d'acide gras insaturé et de monoglycérides d'acide gras saturé, la proportion en monoglycérides d'acide gras saturé étant cependant de préférence inférieure à celle de monoglycérides d'acide gras insaturé.

Dans le cas où l'on utilise des monoglycérides d'acide gras insaturé comme composé (i), les particules de gel cubique contiennent de préférence, comme composé (i), un mélange d'un tel composé et de phytantriol, et plus précisément un mélange comprenant une quantité de phytantriol allant de 1 à 50 % en poids et mieux de 10 à 30 % en poids par rapport au poids total du mélange et une quantité de monoglycéride d'acide gras insaturé allant de 50 à 99 % en poids et mieux de 70 à 90 % en poids par rapport au poids du mélange.

L'agent dispersant et stabilisant (ii) de ce premier mode de réalisation des particules de gel cubique est de préférence choisi parmi :
(1) les alkyl ou alcényl éthers ou esters de polyol,
(2) les amino-acides N-acylés et leurs dérivés, et les peptides N-acylés par un radical alkyle ou alcényle et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

Dans les composés ci-dessus énumérés, les radicaux alkyle et alcényle ont de préférence 8 à 22 atomes de carbone et peuvent se présenter sous forme de mélanges.
(1) Comme alkyl ou alcényl éthers ou esters de polyol, on peut citer notamment :
   (a) les alkyl ou alcényl esters de sorbitan polyoxyéthylénés comportant au moins 20 motifs d'oxyde d'éthylène, tels que le palmitate de sorbitan 20 OE ou Polysorbate 40 commercialisé sous la dénomination de « Montanox 40 DF » par la Société Seppic, et le laurate de sorbitan 20 OE ou Polysorbate 20 commercialisé sous la dénomination de « Tween 20 » par la Société ICI,
   (b) les alkyl ou alcényl esters de polyglycérol comportant au moins 10 motifs dérivés du glycérol, oxyéthylénés ou non, tels que le polyglycéryl-10 laurate commercialisé sous la dénomination de « Decaglyn 1-L » par la Société Nikko Chemicals,
   (c) les alkyl ou alcényl éthers de polyglycérol, tels que le polyglycéryl-3 hydroxylauryléther commercialisé sous la dénomination de « Chimexane NF » par la Société Chimex, et
   (d) les alkyl ou alcényl éthers ou esters de mono ou polysaccharides, tels que ceux dérivant du glucose, du fructose, du galactose, du maltose ou du lactose et notamment les monoesters en 1- et 6- du D-fructose, du décylglucose et du décylpolyglucose.
(2) Comme amino-acides N-acylés et dérivés, et comme peptides N-acylés par un radical alkyle ou alcényle, et sels, on utilise de préférence ceux pour lesquels le radical alkyle ou alcényle comporte au moins 12 atomes de carbone.
   Par amino-acides, on entend selon l'invention les alpha-, bêta- ou gamma-amino-acides. Comme sels d'amino-acides N-acylés, on peut citer par exemple les sels de N-acylglutamate, tels que le cocoylglutamate de monosodium, le lauroylglutamate de monosodium, l'alcoyl C14-C20 glutamate de disodium (le radical alcoyle C14-C20 dérivant du suif hydrogéné), commercialisés respectivement sous les dénominations de « Acylglutamate CS-11 », de « Acylglutamate LS-11 » et de « Acylglutamate HS-21 » par la Société Ajinomoto, ou bien encore le stéaroylglutamate de sodium, commercialisé sous le nom « Acylglutamate HS-11 » par la société Ajinomoto. On peut encore citer les lysines N-acylées telles que la lauroyl-lysine commercialisée sous la dénomination de « Amihope LL » par la Société Ajinomoto. Les dérivés d'amino-acides N-acylés et leurs sels sont de préférence les sarcosinates N-acylés tels que le lauroyl-sarcosinate de sodium commercialisé sous la dénomination de « Oramix L30 » par la Société Seppic, le myristoylsarcosinate de sodium et le palmitoylsarcosinate de sodium commercialisés respectivement sous les dénominations de « Nikkol Sarcosinate MN » et de « Nikkol Sarcosinate PN » par la Société Nikko Chemicals.
   Parmi les peptides N-acylés, on peut citer ceux dérivés de tout ou partie du collagène ou de la kératine tels que le lauroyl collagène de sodium et la palmitoyl kératine commercialisés sous les dénominations de « Proteol B 30 » et de « Lipacide PK » par la Société Seppic.
(3) Parmi les alkyl ou alcényl éthers ou esters sulfates, leurs dérivés et leurs sels, on utilise de préférence ceux pour lesquels le radical alkyle ou alcényle comporte au moins 12 atomes de carbone.
   Parmi les alkyl ou alcényl éthers sulfates, on utilise de préférence les sels d'alkyl éther sulfate et notamment le lauryléther sulfate de sodium. Parmi les alkyl ou alcényl esters sulfates, on peut citer par exemple les esters de l'acide iséthionique ainsi que ses sels et notamment le cocoyl iséthionate de sodium commercialisé sous la dénomination de « Geropon AC 78 » par la Société Rhône Poulenc.
(4) Parmi les alkyl ou alcényl éthers ou esters gras polyoxyéthylénés, on utilise de préférence ceux pour lesquels le radical alkyle ou alcényle comporte au moins 12 atomes de carbone. Ceux particulièrement préférés ont au moins 20 motifs d'oxyde d'éthylène tels que par exemple le PEG-20 stéarate, le laureth-23, l'oleth-20 et le PEG-25 phytostérol.
(5) Parmi les acides alkyl ou alcényl carboxyliques polyoxyéthylénés et leurs sels, on utilise de préférence ceux comportant au moins 10 motifs d'oxyde d'éthylène tels que par exemple l'acide laureth-10 carboxylique et l'acide oleth-10 carboxylique.
(6) Parmi les N-alkyl ou alcényl bétaïnes, on utilise de préférence celles pour lesquelles le radical alkyle ou alcényle comporte au moins 12 atomes de carbone telles que par exemple la lauryl-amidopropyl bétaïne et l'oléyl-amidopropyl bétaïne.
(7) Parmi les alkyl ou alcényl triméthylammonium et leurs sels, on utilise de préférence ceux pour lesquels le radical alkyle ou alcényle comporte au moins 12 atomes de carbone. Comme sels, on utilise de préférence les bromures et chlorures tels que le chlorure de cocoyltriméthylammonium et le bromure de cétyltriméthylammonium.

Lorsque le composé (i) est un dérivé N-2-alcoxycarbonyle de N-méthyl glucamine de formule (I), on utilise de préférence comme agent dispersant et stabilisant (ii), le polyglycéryl-3 hydroxylauryléther, le lauryléthersulfate de sodium ou le bromure de cétyltriméthylammonium.

Selon un second mode de réalisation de l'invention, les particules de gel cubique sont formées par un mélange d'au moins deux composés amphiphiles, l'un des composés amphiphiles étant susceptible de former en présence d'eau une phase lamellaire et l'autre étant susceptible de former en présence d'eau une phase hexagonale inverse.

Le mélange des deux composés amphiphiles formant les particules de gel cubique est caractérisé par le fait qu'aucun des deux composés amphiphiles ne peut conduire par lui-même à une phase cubique lorsqu'il est mis en présence d'eau et que seul leur mélange conduit à une telle phase, et que, par ailleurs, l'un des composés amphiphiles est susceptible de former en présence d'eau une phase lamellaire, tandis que l'autre composé amphiphile est susceptible de former en présence d'eau une phase hexagonale inverse.

On entend par phase lamellaire (phase D selon EKWALL), une phase cristal liquide à symétrie plane, comprenant plusieurs bicouches d'amphiphile disposées en parallèle et séparées par un milieu liquide qui est généralement de l'eau.

On entend par phase hexagonale inverse (phase F selon EKWALL), une phase cristal liquide correspondant à une arrangement hexagonal de cylindres parallèles remplis d'un milieu liquide qui est généralement de l'eau, séparés par un environnement hydrocarboné correspondant aux chaînes grasses de l'amphiphile.

Une définition plus précise de ces dénominations est donnée dans Ekwall (1968), Adv . Liq. Cryst. (Brown G.H., Ed.), Chap. 1, 14. Chacune de ces phases possède une texture caractéristique au microscope en lumière polarisée, dont on pourra trouver une description plus précise dans Rosevear (1968), JSCC, 19, 581. et dans Lachampt et Vila (1969), Revue Française des Corps Gras, n°2, 87-111.

De manière préférée, le composé amphiphile susceptible de former une phase lamellaire est choisi parmi les monoesters de diglycérol, tels que l'isostéarate de diglycérol (Solvay) et le mono-oléate de diglycérol (RYLO PG 29^{R} commercialisé par la société Danisco), seuls ou en mélange.

Le composé amphiphile susceptible de former une phase hexagonale inverse est de préférence choisi parmi les mono-, di- ou tri-esters de diglycérol, les carbamates d'aminopolyols et leurs mélanges. Comme mono-, di- ou tri-esters de diglycérol, on peut citer par exemple le décyl-2 tétradécanoate de diglycérol et le di/tri-oléate de diglycérol (TSED 396^{R} commercialisé par la société Danisco). Comme carbamates d'aminopolyols, on peut citer entre autres le 3-N-(2-décyl-tétradécyloxycarbonyl)-amino-1,2-propanediol et le N-2-dodécylhexadécyloxycarbonyl-N-méthyl-D-glucamine. Ces carbamates d'aminopolyols sont décrits dans le document EP-A-666251 cité ici pour référence.

De préférence, le mélange des deux types de composés amphiphiles est constitué de 10 à 90 % en poids et mieux 15 à 85 % en poids d'au moins un composé amphiphile susceptible de former une phase lamellaire, et de 10 à 90 % en poids et mieux de 15 à 85 % en poids d'au moins un composé amphiphile susceptible de former une phase hexagonale inverse, par rapport au poids total du mélange.

Le rapport entre les deux types de composés amphiphiles dépend des composés utilisés et l'homme du métier saura déterminer la quantité de chaque type de composé à utiliser afin d'obtenir des particules de gels cubiques.

Plus précisément, les mélanges constituant les particules de gel cubique des compositions de l'invention sont de préférence réalisés à l'aide des associations suivantes :
- 55 à 75 % en poids d'isostéarate de diglycérol et 25 à 45 % en poids de décyl-2 tétradécanoate de diglycérol ;
- 30 à 65 % en poids d'isostéarate de diglycérol et 35 à 70 % en poids de di/tri-oléate de diglycérol ;
- 75 à 85 % en poids d'isostéarate de diglycérol et 15 à 25 % en poids de 3-N-(2-décyltétradécyloxycarbonyl)-amino-1,2-propanediol ;
- 55 à 75 % en poids d'isostéarate de diglycérol et 25 à 45 % en poids de N-2-dodécylhexadécyl-oxycarbonyl-N-méthyl-D-glucamine ;
- 15 à 50 % en poids de mono-oléate de diglycérol et 50 à 85 % en poids de di/tri-oléate de diglycérol.

Dans ce second mode de réalisation, le mélange des composés constituant les particules de gel cubique est de préférence réalisé en dispersion aqueuse, et de préférence en présence d'au moins un agent dispersant et stabilisant, celui-ci pouvant être en particulier choisi parmi les composés (ii) indiqués ci-dessus pour le premier mode de réalisation des particules de gel cubique. Quand il est présent, l'agent dispersant et stabilisant est utilisé en une quantité allant par exemple d'environ 0,1 à 3 % en poids par rapport au poids total de la dispersion.

Dans les deux modes de réalisation des particules de gel cubique, décrits ci-dessus, on peut ajouter dans la dispersion aqueuse contenant ces particules, un lipide amphiphile ionique non hydrosoluble, de préférence en une quantité allant de 0,0005 % à 5 % en poids et mieux de 0,001 % à 2 % en poids par rapport au poids total de la dispersion. Parmi les lipides amphiphiles ioniques non hydrosolubles, on peut notamment citer :
(i) les phospholipides tels que les phospholipides naturels comme la lécithine de soja ou d'oeuf, les phospholipides modifiés par voie chimique ou enzymatique comme la lécithine hydrogénée ou le sel de sodium de l'acide phosphatidique, et les phospholipides de synthèse comme la dipalmitoylphosphatidylcholine,
(ii) les esters phosphoriques d'acide gras et leurs sels, notamment de sodium et de potassium, tels que le monocétyl phosphate commercialisé sous la dénomination de "Monafax 160/" par la Société Mona, ainsi que le dimyristyl phosphate commercialisé sous la dénomination de « Mexoryl SY » par la Société Chimex,
(iii) les dérivés N-acylés de l'acide glutamique et leurs sels, tels que le stéaroylglutamate de monosodium commercialisé sous la dénomination de « Acylglutamate HS 11 » par la Société Ajinomoto, et le mélange cocoyl-alcoyl C14-C20 glutamate de monosodium, le radical alcoyle C14-C20 dérivant du suif hydrogéné, commercialisé sous la dénomination de « Acylglutamate GS 11 » par la Société Ajinomoto,
(iv) le cétylsulfate de sodium commercialisé sous la dénomination de « Nikkol SCS » par la Société Nikko Chemicals,
(v) le cocoyl monoglycéride sulfate de sodium commercialisé sous la dénomination de « Nikkol SGC 80 N » par la Société Nikko Chemicals, et
(vi) les dérivés d'ammonium quaternaire non hydrosolubles tels que le chlorure de béhényltriméthylammonium, le chlorure de dilauryldiméthylammonium, le chlorure de distéaryl-diméthylammonium, le méthylsulfate de 4,5-dihydro 1-méthyl 2-alcoyl C14-C20 1-(2-alcoyl C14-C20 aminoéthyl)imidazolium, les radicaux alcoyles C14-C20 dérivant du suif hydrogéné, commercialisé sous la dénomination de « Rewoquat W75H » par la Société Rewo Chemische, le méthylsulfate de dialkylhydroxyéthylméthylammonium dont les radicaux alkyles dérivent du suif, hydrogéné ou non, commercialisé sous la dénomination de « Stepanquat VP 85 » par la Société Stepan et le « Quatemium-82 » commercialisé par la Société Seppic sous la dénomination de « Amonyl DM ».

L'incorporation de ces lipides amphiphiles ioniques non hydrosolubles confère aux particules de gel cubique une charge superficielle entraînant une répulsion électrostatique des particules entre elles.

Les particules de gel cubique, telles que définies précédemment, ont une taille qui peut être modulée par la nature et la concentration des composés les constituant. Généralement, ces particules ont une taille moyenne en nombre, mesurée à l'aide d'un granulomètre laser BI 90 de la Société Brookhaven Instruments Corporation, d'environ 0,05 µm à environ 1 µm, et de préférence inférieure ou égale à 0,5 µm.

Il est en outre possible d'incorporer dans les particules de gel cubique, divers types de composés actifs. En particulier, lesdites particules peuvent contenir un principe actif hydrophile ou lipophile. Bien entendu, grâce à la structure particulière des particules de gel cubique, il est possible d'incorporer dans celles-ci à la fois des principes actifs hydrophiles et lipophiles, même si ceux-ci présentent une certaine incompatibilité.

Les compositions utilisées selon l'invention peuvent constituer notamment des compositions cosmétiques et dermatologiques. Elles contiennent pour une telle application, un milieu physiologiquement acceptable. On entend ici par « milieu physiologiquement acceptable », un milieu compatible avec la peau, et éventuellement les lèvres, le cuir chevelu, les cils, les yeux et/ou les cheveux. Ce milieu physiologiquement acceptable peut être plus particulièrement constitué d'eau et éventuellement d'un solvant organique physiologiquement acceptable choisi par exemple parmi les alcools inférieurs comportant de 1 à 4 atomes de carbone comme l'éthanol, l'isopropanol, le propanol, le butanol ; les polyéthylène glycols ayant de 6 à 80 motifs oxyde d'éthylène ; les polyols comme le propylène glycol, l'isoprène glycol, le butylène glycol, la glycérine, le sorbitol. Le milieu physiologiquement acceptable de la composition selon l'invention a un pH compatible avec la peau, et de préférence allant de 3 à 8 et mieux de 5 à 7.

Selon un mode préféré de réalisation, les compositions utilisées dans la présente invention comportent en outre une phase huileuse, apportant notamment du confort et de la douceur lors de l'application sur la peau. La quantité de phase huileuse peut aller par exemple de 2 à 40 % en poids et de préférence de 5 à 25 % en poids par rapport au poids total de la composition, le reste de la composition étant constitué de la phase aqueuse contenant le phytantriol ou constituée par les particules de gel cubique contenant le phytantriol ou contenant la dispersion aqueuse des particules de gel cubique contenant le phytantriol. Quand le phytantriol est dans des particules de gel cubique, le rapport en poids des composés amphiphiles constituant les particules de la phase cubique et de la phase huileuse va de préférence de 0,02/1 à 1/1, et mieux de 0,05/1 à 0,5/1.

Comme huiles utilisables dans l'invention, on peut citer les huiles minérales (huile de vaseline), les huiles végétales (fraction liquide du beurre de karité, huile de tournesol, huile d'amande d'abricot), les huiles animales (perhydrosqualène), les huiles de synthèse (polyisobutène hydrogénée, néopentanoate d'isostéaryle, myristate d'isopropyle), les huiles de silicone non volatiles ou volatiles (cyclométhicones telles que cyclopentasiloxane et cyclohexasiloxane) et les huiles fluorées (perfluoropolyéthers). On peut aussi utiliser, comme matières grasses, des alcools gras, des acides gras, des cires. La phase huileuse de l'émulsion peut contenir aussi des gommes telles que les gommes de silicone, des résines et des cires.

La composition contenant une phase huileuse peut être sous forme d'une émulsion eau-dans-huile (E/H) ou huile-dans-eau (H/E). Selon un mode préféré de réalisation, elle est sous forme d'une émulsion huile-dans-eau.

De façon connue, les compositions de l'invention peuvent contenir également des adjuvants habituels dans les domaines cosmétique ou dermatologique, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur, les matières colorantes, les sels. Les quantités de ces différents adjuvants sont celles classiquement utilisées dans le domaine considéré, et par exemple de 0,01 à 20 % du poids total de la composition, et de préférence de 0,01 à 10 % du poids total de la composition. Ces adjuvants, selon leur nature, peuvent être introduits dans la phase grasse, dans la phase aqueuse et/ou dans des sphérules lipidiques.

Comme actifs, la composition peut contenir des actifs classiquement utilisés en cosmétique, tels que les agents empêchant l'adhésion des bactéries à la surface de la peau, les agents desquamants, les agents hydratants, les agents anti-séborrhéiques, les agents dépigmentants ou pro-pigmentants, les agents anti-glycation, les inhibiteurs de NO-synthase, les inhibiteurs de 5α-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents tenseurs, les agents antipollution ou anti-radicalaires, les agents apaisants, les actifs lipolytiques ou ayant une activité favorable, directe ou indirecte, sur la diminution du tissu adipeux, les agents agissant sur la microcirculation, et les agents agissant sur le métabolisme énergétique des cellules.

De façon avantageuse, outre les particules de gel cubique telles que définie précédemment, la composition peut contenir d'autres agents empêchant l'adhésion des bactéries à la surface de la peau tels que les huiles et les corps gras décrits dans la demande de brevet EP-1 313 086, ou les huiles végétales alcoxylées décrite dans la demande de brevet FR-2 832 057. Elle peut aussi avantageusement contenir d'autres actifs choisis parmi les agents desquamants, les agents hydratants, les agents anti-séborrhéiques et leurs mélanges.

Selon un autre mode de réalisation de l'invention, les compositions utilisées peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques préférés sont choisis parmi l'éthylhexyl salicylate, l'éthylhexyl methoxycinnamate, l'octocrylene, le phenylbenzimidazole sulfonic acid, la benzophenone-3, la benzophenone-4, la benzophenone-5, la 4-methylbenzylidene camphor, le terephthalylidene dicamphor sulfonic acid, le disodium phenyl dibenzimidazole tetra-sulfonate, la 2,4,6-tris-(4'-amino benzalmalonate de diisobutyle)-s-triazine, l'anisotriazine, l'éthylhexyl triazone, la diethylhexyl butamido triazone, le methylène bis-benzotriazolyl tetramethylbutylphénol, le drometrizole trisiloxane, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

Comme gélifiants, on peut citer par exemple les dérivés de cellulose tels que l'hydroxyéthylcellulose et les alkylhydroxyéthylcelluloses telles que la cétylhydroxyéthylcellulose ; les dérivés d'algues tels que le satiagum ; les gommes naturelles telles que l'adragante ou la gomme guar ; les polymères synthétiques tels que les polymères ou copolymères carboxyvinyliques et en particulier ceux commercialisés sous les dénominations de Carbopol^{R} par la société Goodrich ou de Synthalen^{R} par la société 3V SA. La proportion en agent gélifiant va de préférence de 0,1 à 2 % du poids total de la composition.

Les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

De préférence, les compositions utilisées selon l'invention sont obtenues selon un procédé de préparation comprenant au moins deux étapes. La première étape consiste généralement à préparer une dispersion aqueuse de particules de gel cubique, telles que définies précédemment, par fragmentation, à l'aide d'un homogénéiseur, des composés tels que définis précédemment et d'eau, en présence éventuellement de lipides amphiphiles ioniques non hydrosolubles et/ou de principes actifs hydrophiles et/ou lipophiles et/ou d'un agent dispersant et stabilisant tels que définis précédemment. L'homogénéiseur peut être du type rotor-stator à fort gradient de cisaillement comme le Virtis^{R} ou le Heidolph Diax 600^{R} ou un homogénéiseur haute pression fonctionnant entre 200 et 1.800 bars environ (20 à 180 MPa).

Il est bien entendu possible d'introduire, à ce stade de la préparation de la dispersion aqueuse des particules de gel cubique, divers additifs et/ou principes actifs dans la phase aqueuse. Après la formation des particules de gel cubique, l'agent dispersant et stabilisant se trouve, généralement, à l'extérieur desdites particules.

La deuxième étape consiste généralement ensuite à ajouter à ladite dispersion obtenue une phase huileuse contenant éventuellement certains additifs et/ou principes actifs lipophiles et à soumettre le mélange à une agitation mécanique qui peut être notamment réalisée à l'aide d'un homogénéiseur du même type que ceux définis ci-dessus.

Divers additifs et/ou principes actifs peuvent être également introduits à ce stade de la préparation. Par ailleurs, lorsque l'on souhaite préparer une dispersion gélifiée, dans une troisième étape, on ajoute généralement au mélange obtenu après la seconde étape, une solution aqueuse contenant un agent gélifiant.

L'utilisation de particules de gel cubique selon l'invention trouve une application plus spécifiquement dans le domaine cosmétique ou dermatologique. En particulier, les compositions contenant les particules de gel cubique pourront être utilisées pour le nettoyage et/ou le démaquillage et/ou le soin de la peau. Les particules de gel cubique peuvent aussi être utilisées dans le cadre de la présent invention dans des produits solaires, dans des produits de maquillage comme les fonds de teint, dans les rouges à lèvres, les mascaras, les fards, et/ou dans les déodorants.

La présente invention concerne également un procédé cosmétique non thérapeutique pour prévenir et/ou lutter contre les désordres liés à l'adhésion des micoorganismes comprenant l'application sur la peau et/ou les muqueuses d'une composition selon l'invention, contenant des particules de gel cubique dans un milieu physiologiquement acceptable.

Plus spécifiquement l'invention concerne un procédé de traitement cosmétique non thérapeutique des peaux grasses comprenant l'application topique d'une composition selon l'invention, contenant des particules de gel cubique dans un milieu physiologiquement acceptable.

Un aspect particulier de l'invention concerne l'utilisation des particules de gel cubique, en tant qu'agent empêchant ou réduisant l'adhésion des microorganismes sur la surface de la peau et/ou des muqueuses dans une composition démaquillante de la peau.

Ainsi un autre objet de la présente invention concerne un procédé cosmétique non thérapeutique de nettoyage et/ou de démaquillage de la peau comprenant l'application topique d'une composition contenant des particules de gel cubique selon l'invention, dans un milieu physiologiquement acceptable.

La flore microbienne de la surface de la peau étant responsable d'un grand nombre de désordres allant du simple désagrément (odeur, boutons, ... ) aux maladies plus graves, la présente invention a aussi pour objet l'utilisation cosmétique des particules de gel cubique, en tant qu'agent destiné à diminuer les mauvaises odeurs corporelles, et/ou destiné aux soins d'hygiène corporelle.

Un autre aspect de l'invention concerne donc un procédé cosmétique non thérapeutique pour réduire les mauvaises odeurs corporelles comprenant l'application topique d'une composition selon l'invention contenant des particules de gel cubique, dans un milieu physiologiquement acceptable.

La présente invention a également pour objet l'utilisation des particules de gel cubique pour la préparation d'une composition dermatologique destinée à prévenir et/ou lutter contre l'acné, particulièrement l'acné juvénile.

La présente invention a aussi pour objet l'utilisation des particules de gel cubique pour la préparation d'une composition dermatologique destinée à prévenir et/ou lutter contre les mycoses.

Par "*destiné aux soins d'hygiène corporelle*" on entend une substance destinée à être mise en contact avec les diverses parties superficielles du corps humain et/ou avec la muqueuses, et/ou avec les dents, en vue de les nettoyer, de les protéger, de les maintenir en bon état, d'en modifier l'aspect, de les parfumer, ou d'en corriger l'odeur.

Les exemples qui suivent servent à illustrer l'invention sans toutefois présenter un caractère limitatif. Les composés sont, selon le cas, cités en noms chimiques ou en noms CTFA (International Cosmetic Ingredient Dictionary and Handbook).

### Exemple 1 :

| | | |
|---|---|---|
| Phase A | Phytantriol | 2,97 % |
| | Stéaroyle glutamate de sodium | 0,03 % |
| | Eau | 1,6 % |
| Phase B | Polysorbate 40 | 1 % |
| | Glycérine | 4 % |
| | Eau | 46,15 % |
| Phase C | Mélange de caprylate et heptanoate de stéaryle | 2,5 % |
| | Isohexadécane | 2,5 % |
| | Huile d'abricot | 5 % |
| | Stéarate d'isocétyle | 3 % |
| | Cyclohexasiloxane | 6 % |
| | Filtre UV | 1 % |
| | Parfum | 0,2 % |
| Phase D | Hydroxyéthylcéllulose | 1 % |
| | Pentasodium éthylènediamine tétraméthylène | |
| | Phosphonate | 0,033 % |
| | Eau | 23,017 % |

### Mode opératoire :

Le mélange des phases A et B est placé sous agitation à 60-70°C jusqu'à homogénéisation, puis refroidi à 25°C.
La phase C est ensuite chauffée vers 50°C et homogénéisée à l'aide d'un agitateur magnétique, puis refroidie à température ambiante.
La phase C est ensuite ajoutée au mélange A et B à température ambiante sous agitation pendant 15 mn.
Le mélange obtenu est ensuite passé deux fois à l'homogénéisateur à Haute Pression. (600 bars à température ambiante).

La préparation obtenue est alors gélifiée à l'aide du mélange de la phase D (préalablement gonflée sous Rayneri à température ambiante.

On obtient alors une crème homogène qui, après application sur la peau, empêche l'adhésion des bactéries à la surface de la peau.

Cette crème est appropriée pour le traitement des mycoses et/ou de l'acné.

### Exemple 2 : Test d'anti-adhésion microbienne :

### Protocole :

L'activité des particules de gel cubique utilisées selon l'invention a été mise en évidence sur épiderme reconstruit.
Avant l'adhésion bactérienne, l'épiderme reconstruit est mis en contact pendant 2 heures avec la composition de l'exemple 1 à 37°C. On y ajoute alors 1 ml de suspension bactérienne de *Staphylococcus aureus* à une concentration de 10⁷ germes/ml dans du Tryptone sel. Après incubation 24 heures à 37°C, la suspension bactérienne est vidée et cinq rinçages sont réalisés par 1 ml d'eau distillée stérile. L'épiderme reconstruit détaché de son support est alors broyé à l'aide d'un robot dans 18 ml de Tryptone sel. On effectue une dilution décimale de cette suspension dans le Tryptone sel, on procède ensuite à un ensemencement de 1 ml de la dilution dans 15 ml de la gélose Trypticase Soja et à l'incubation pendant 24 heures à 37°C. On dénombre ensuite les cellules adhérentes et viables.

Ce test d'anti-adhésion permet d'évaluer l'efficacité de molécules seules ou de produits finis.

Avant le test d'anti-adhésion, on met en oeuvre le test de viabilité suivant :

Un mélange bactéries/produit à tester, dans le même rapport que dans le test anti-adhésion, est mis en contact 24 heures à 37°C. Le dénombrement des germes est réalisé par dilution décimale dans du Tryptone sel et ensemencement au râteau de 100 µl sur de la gélose Trypticase Soja. Le comptage des colonies s'effectue après 24 heures d'incubation à 37°C.

L'essai de viabilité réalisé préalablement au test d'anti-adhésion permet d'écarter toute composante bactéricide des molécules ou des produits finis testés et de ne mettre en évidence que l'activité anti-adhésion.

### Résultats :

Les résultats obtenus sont récapitulés dans le tableau suivant :

| **Formule testée** | **Activité anti-adhésion** | |
|---|---|---|
| | Résultats quantitatifs | Résultats qualitatifs |
| Composition de l'exemple 1 | -1,7 | Bonne |

Les résultats quantitatifs correspondent à la diminution du logarithme décimal du nombre moyen de *Staphylococcus aureus* viable adhérent sur l'épiderme reconstruit après traitement par la formule testée par rapport au logarithme décimal du nombre moyen de *Staphylococcus aureus* viable adhérent sur l'épiderme reconstruit après traitement à l'eau dans les mêmes conditions. Les résultats sont considérés comme significatifs si la différence est supérieure à 0,5 log.
Les résultats qualitatifs sont exprimés par un terme variant en fonction des valeurs de Log de réduction de l'adhésion du germes au bout de 24 heures par rapport au même test utilisant l'eau :
- résultats supérieurs à ceux obtenus avec l'eau Pro-adhésion.
- résultats identiques à ceux obtenus avec l'eau Sans effet,
- entre 0.5 et 1 log de réduction par rapport à l'eau Faible,
- entre 1 et 1.5 log de réduction par rapport à l'eau Moyenne,
- 1.5 et 2 log de réduction par rapport à l'eau Bonne,
- 2 Log et plus de réduction par rapport à l'eau Excellente,

## Revendications

1. Utilisation cosmétique non thérapeutique de particules de gel cubique dans une composition en tant qu'agent empêchant ou réduisant l'adhésion des microorganismes sur la surface de la peau et/ou des muqueuses.

2. Utilisation de particules de gel cubique pour la préparation d'une composition dermatologique destinée prévenir ou lutter contre les pathologies liées à l'adhésion des microorganismes sur la surface de la peau et/ou des muqueuses.

3. Utilisation selon l'une des revendications 1 ou 2 **caractérisée en ce que** les particules de gel cubique sont en dispersion aqueuse.

4. Utilisation selon l'une des revendications 1 à 3 **caractérisée en ce que** les particules de gel cubique sont formées par un mélange comprenant (i) de 0,1 à 15 % en poids par rapport au poids total de la composition, d'au moins un composé choisi parmi le 3,7,11,15-tétraméthyl-1,2,3-hexadecanetriol ou phytantriol, les dérivés N-2-alcoxycarbonyles de N-méthylglucamine et les monoglycérides d'acide gras insaturé, et (ii) de 0,05 à 3 % en poids par rapport au poids total de la composition, d'au moins un agent dispersant et stabilisant, ledit agent étant choisi parmi les agents tensioactifs hydrosolubles à température ambiante, contenant une chaîne grasse, saturée ou insaturée, linéaire ou ramifiée, ayant de 8 à 22 atomes de carbone.

5. Utilisation selon la revendication 4 **caractérisée en ce que** la proportion pondérale relative en composé (i) par rapport au poids à l'agent dispersant et stabilisant (ii) est comprise ente 2 et 200.

6. Utilisation selon l'une des revendications 4 ou 5 **caractérisée par le fait que** le dérivé N-2-alcoxycarbonyle de N-méthylglucamine répond à la formule (I) suivante : 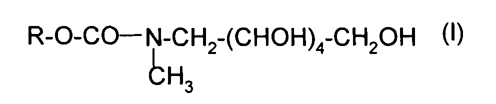 dans laquelle R représente un radical alkyle ramifié comportant de 6 à 18 atomes de carbone.

7. Utilisation selon l'une des revendications 4 à 6 **caractérisée en ce que** le dérivé N-2-alcoxycarbonyles de N-méthylglucamine est choisi parmi la N-2-hexyldécyloxycarbonyl-N-méthyl-glucamine, la N-2-éthylhexyloxycarbonyl-N-méthyl-glucamine, la N-2-butyloctyloxycarbonyl-N-méthyl-glucamine et leurs mélanges.

8. Utilisation selon l'une quelconque des revendications 4 à 7, **caractérisée par le fait que** les particules de gel cubique contiennent comme composé (i), un mélange constitué de 1 à 40 % en poids de phytantriol par rapport au poids du mélange et de 60 à 99 % en poids de dérivé N-2-alcoxycarbonyle de N-méthyl-glucamine par rapport au poids du mélange.

9. Utilisation selon la revendication 4, **caractérisée par le fait que** ledit monoglycéride d'acide gras insaturé est choisi parmi le monooléate de glycéryle et le monolinoléate de glycéryle.

10. Utilisation selon la revendication 4, **caractérisée par le fait que** les particules de gel cubique contiennent comme composé (i) un mélange constitué de 1 à 50 % en poids de phytantriol par rapport au poids du mélange, et de 50 à 99 % en poids de monoglycéride d'acide gras insaturé par rapport au poids du mélange.

11. Utilisation selon l'une quelconque des revendications 4 à 10, **caractérisée par le fait que** ledit agent dispersant et stabilisant est choisi parmi :
(1) les alkyl ou alcényl éthers ou esters de polyol,
(2) les amino-acides N-acylés et leurs dérivés et les peptides N-acylés par un radical alkyle ou alcényle, et leurs sels,
(3) les alkyl ou alcényl éthers ou esters sulfates, leurs dérivés et leurs sels,
(4) les alkyl ou alcényl éthers ou esters gras polyoxyéthylénés,
(5) les acides alkyl ou alcényl carboxyliques polyoxyéthylénés et leurs sels,
(6) les N-alkyl ou alcényl bétaïnes,
(7) les alkyl ou alcényl triméthylammonium et leurs sels, et
(8) leurs mélanges.

12. Utilisation selon l'une quelconque des revendications 1 à 3, **caractérisée par le fait que** les particules de gel cubique sont formées par un mélange d'au moins deux composés amphiphiles, l'un des composés amphiphiles étant susceptible de former en présence d'eau une phase lamellaire et l'autre étant susceptible de former en présence d'eau une phase hexagonale inverse.

13. Utilisation selon la revendication 12, **caractérisée par le fait que** le composé amphiphile susceptible de former une phase lamellaire est choisi parmi les monoesters de diglycérol.

14. Utilisation selon la revendication 12 ou 13, **caractérisée par le fait que** le composé amphiphile susceptible de former une phase hexagonale inverse est choisi parmi les mono-, di- ou tri-esters de diglycérol, les carbamates d'aminopolyols et leurs mélanges.

15. Utilisation selon l'une quelconque des revendications 12 à 14, **caractérisée par le fait que** le composé amphiphile susceptible de former une phase lamellaire est choisi parmi l'isostéarate de diglycérol, le mono-oléate de diglycérol et leurs mélanges.

16. Utilisation selon l'une quelconque des revendications 12 à 15, **caractérisée par le fait que** le composé amphiphile susceptible de former une phase hexagonale inverse est choisi parmi le décyl-2 tétradécanoate de diglycerol, le di/tri-oléate de diglycérol, le 3-N-(2-décyltétradécyloxycarbonyl)-amino-1,2-propanediol et le N-2-dodécylhexadécyl-oxycarbonyl-N-méthyl-D-glucamine, et leurs mélanges.

17. Utilisation selon l'une quelconque des revendications 12 à 16, **caractérisée par le fait que** le mélange des deux composés amphiphiles est constitué de 10 à 90 % en poids du composé amphiphile susceptible de former une phase lamellaire et de 10 à 90 % en poids du composé amphiphile susceptible de former une phase hexagonale inverse, par rapport au poids total du mélange.

18. Utilisation selon l'une quelconque des revendications 12 à 17, **caractérisée par le fait que** le mélange des deux composés amphiphiles est choisi parmi les mélanges suivants :
- 55 à 75 % en poids d'isostéarate de diglycérol et 25 à 45 % en poids de décyl-2 tétradécanoate de diglycérol ;
- 30 à 65 % en poids d'isostéarate de diglycérol et 35 à 70 % en poids de di/tri-oléate de diglycérol;
- 75 à 85 % en poids d'isostéarate de diglycérol et 15 à 25 % en poids de 3-N-(2-décyltétradécyloxycarbonyl)-amino-1,2-propanediol ;
- 55 à 75 % en poids d'isostéarate de diglycérol et 25 à 45 % en poids de N-2-dodécylhexadécyl-oxycarbonyl-N-méthyl-D-glucamine ;
- 15 à 50 % en poids de mono-oléate de diglycérol et 50 à 85 % en poids de di/tri-oléate de diglycérol.

19. Utilisation selon l'une quelconque des revendications 1 à 18, **caractérisée par le fait que** les particules de gel cubique ont une taille allant de 0,05 µm à 1 µm.

20. Utilisation selon l'une quelconque des revendications 3 à 19, **caractérisée par le fait que** la dispersion de particules de gel cubique comprend en outre au moins un lipide amphiphile ionique non hydrosoluble.

21. Utilisation selon la revendication 20, **caractérisée par le fait que** ledit lipide amphiphile ionique non hydrosoluble est choisi parmi :
(i) les phospholipides,
(ii) les esters phosphoriques d'acide gras,
(iii) les dérivés N-acylés de l'acide glutamique non hydrosolubles et leurs sels,
(iv) le cétylsulfate de sodium,
(v) le cocoyl monoglycéride sulfate de sodium, et
(vi) les dérivés d'ammonium quaternaire non hydrosolubles.

22. Utilisation selon l'une quelconque des revendications 1 à 21, **caractérisée par le fait que** les particules comprennent au moins un principe actif hydrophile et/ou lipophile.

23. Utilisation selon l'une quelconque des revendications 1 à 22, **caractérisée par le fait que** les particules de gel cubique sont présentes en une quantité allant de 0,1 à 20 % en poids par rapport au poids total de la composition.

24. Utilisation selon l'une des revendications 1 à 23 **caractérisée en ce que** les compositions utilisées comportent en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

25. Procédé cosmétique non thérapeutique de nettoyage et/ou de démaquillage de la peau comprenant l'application topique d'une composition contenant des particules de gel cubique telles que définies dans l'une des revendications 1 à 22, dans un milieu physiologiquement acceptable.

26. Procédé cosmétique non thérapeutique pour prévenir et/ou lutter contre les désordres liés à l'adhésion des microorganismes comprenant l'application sur la peau et/ou les muqueuses d'une composition contenant des particules de gel cubique telles que définies dans l'une des revendications 1 à 22, dans un milieu physiologiquement acceptable.

27. Procédé de traitement cosmétique non thérapeutique des peaux grasses comprenant l'application topique d'une composition contenant des particules de gel cubique telles que définies dans l'une des revendications 1 à 22, dans un milieu physiologiquement acceptable.

28. Procédé cosmétique non thérapeutique pour réduire les mauvaises odeurs corporelles comprenant l'application topique d'une composition contenant des particules de gel cubique telles que définies dans l'une des revendications 1 à 22, dans un milieu physiologiquement acceptable.

29. Utilisation de particules de gel cubique telles que définies dans l'une des revendications 1 à 22, pour la préparation d'une composition dermatologique destinée à prévenir et/ou lutter contre l'acné, particulièrement l'acné juvénile.

30. Utilisation de particules de gel cubique telles que définies dans l'une des revendications 1 à 22, pour la préparation d'une composition dermatologique destinée à prévenir et/ou lutter contre les mycoses.
